# EUROPÄISCHE PATENTSCHRIFT

(11) **EP 3 062 680 B1**
(45) Veröffentlichungstag und Bekanntmachung des Hinweises auf die Patenterteilung: **07.03.2018**
(21) Anmeldenummer: 14790580.6
(22) Anmeldetag: 28.10.2014
(51) Int. Cl.: A61B 1/00, A61B 1/005

(54) **AUSLENKBEWEGUNGSÜBERTRAGUNGSEINRICHTUNG, ENDOSKOPBIEGESTEUERUNG UND ENDOSKOP**
DEFLECTION MOVEMENT TRANSMISSION DEVICE, ENDOSCOPE BEND CONTROL, AND ENDOSCOPE
SYSTÈME DE TRANSMISSION D'UN MOUVEMENT DE DÉVIATION, COMMANDE DE LA COURBURE D'UN ENDOSCOPE ET ENDOSCOPE

(30) Priorität: 30.10.2013 DE 102013222041
(43) Veröffentlichungstag der Anmeldung: 07.09.2016
(73) Patentinhaber: Digital Endoscopy GmbH, 86316 Friedberg (DE)
(72) Erfinder: VIEBACH, Thomas, 86579 Waidhofen (DE); PAUKER, Friedrich, 86420 Diedorf (DE)
(74) Vertreter: TBK
(86) Internationale Anmeldenummer: PCT/EP2014/073065
(87) Internationale Veröffentlichungsnummer: WO 2015/063052

(56) Entgegenhaltungen:
- ES-T3- 2 356 497
- JP-A- 2009 505 688
- JP-A- 2009 530 051
- JP-A- 2012 245 058
- US-A- 3 605 725
- US-A1- 2012 209 068

## Beschreibung

Die vorliegende Erfindung bezieht sich auf eine Auslenkbewegungsübertragungseinrichtung, die eine durch ein Steuerelement bewerkstelligte Auslenkbewegung zu einem Reaktionselement überträgt. Außerdem bezieht sich die vorliegende Erfindung auf eine Endoskopbiegesteuerung und ein Endoskop.

Bei einer solchen Auslenkbewegungsübertragungseinrichtung wird eine Schwenkbewegung eines Steuerelementes in eine Auslenkbewegung eines Auslenkelementes umgewandelt. Solche Auslenkbewegungsübertragungseinrichtungen sind vielseitig anwendbar.

Ein Anwendungsgebiet der Auslenkbewegungsübertragungseinrichtung ist ein Endoskop, bei dem ein biegbares Ende eines Katheters, das heißt ein sogenannter Deflectingabschnitt, durch Schwenken eines Steuerelementes bewegt wird, wobei die Bewegung des Deflectingabschnittes genau der Bewegung des Steuerelementes folgt.

Bei medizinischen Untersuchungen mit einem Endoskop sollte die Übertragung einer Schwenkbewegung eines Steuerelementes zu einer Biegebewegung des Deflectingabschnittes möglichst genau sein. Dokument ES 2356 497 zeigt eine Auslenkbewegungsübertragungseinrichtung nach dem Oberbegriff des Anspruchs 1.

### DURCH DIE ERFINDUNG ZU LÖSENDE AUFGABE

Aufgabe der vorliegenden Erfindung ist es, eine verbesserte Auslenkbewegungsübertragungseinrichtung zu schaffen.

Insbesondere ist es die Aufgabe der vorliegenden Erfindung, eine Auslenkbewegungsübertragungseinrichtung mit besonders günstiger Funktionalität und einfacher Handhabung zu schaffen. Außerdem sollen eine verbesserte Endoskopbiegesteuerung und ein verbessertes Endoskop geschaffen werden.

### LÖSUNG DER AUFGABE

Diese Aufgabe wird erfindungsgemäß durch eine Auslenkbewegungsübertragungseinrichtung mit den Merkmalen von Anspruch 1 gelöst. Vorteilhafte Weiterbildungen sind Gegenstand der abhängigen Ansprüche. Eine Endoskopbiegesteuerung ist in Anspruch 10 aufgezeigt, und ein Endoskop ist in Anspruch 11 aufgezeigt.

Die Erfindung betrifft somit eine Auslenkbewegungsübertragungseinrichtung mit einem Steuerelement zur Bewerkstelligung einer Auslenkbewegung, wobei das Steuerelement einen Schwenkabschnitt besitzt, der an einem Kopfabschnitt eines Basiselements abgestützt ist und zur Bewerkstelligung einer Auslenkbewegung relativ zum Kopfabschnitt des Basiselements ist; zumindest einem Bewegungsübertragungsdrahtkörper, der am Schwenkabschnitt des Steuerelements angelenkt ist; einem länglichen Übertragungsführungskörper, in dessen Längsrichtung der Bewegungsübertragungsdrahtkörper geführt ist; und einem auszulenkenden biegbaren Körper, der am vom Steuerelement entgegengesetzten Ende des Übertragungsführungskörpers sitzt und an dem der Bewegungsübertragungsdrahtkörper beabstandet von der Verbindung zum Übertragungsführungskörper angebracht ist.

Diese Auslenkbewegungsübertragungseinrichtung hat an einem Ende ein Steuerelement. Eine Schwenkbewegung des Steuerelements wird über Bewegungsübertragungsdrahtkörper zu einem auszulenkenden biegbaren Körper am anderen Ende der Auslenkbewegungsübertragungseinrichtung übertragen. Die Schwenkbewegung des Steuerelements erfolgt relativ zum Basiselement. Dabei stehen bei der Schwenkbewegung des Steuerelements die bei der Schwenkbewegung involvierten Elemente, d.h. das Steuerelement und das Basiselement miteinander in Kontakt. Zu Beginn, während und nach der Schwenkbewegung des Steuerelements ist somit ein Abstützen des Steuerelements am Basiselement gewährleistet. Dies stellt eine exakt definierte Schwenkbewegung des Steuerelements sicher.

Der Bewegungsübertragungsdrahtkörper ist dabei an einer Position am Außenumfang des Schwenkabschnittes des Steuerelements angelenkt, die bei einer Schwenkbewegung des Steuerelements ihren Abstand zum Kopfabschnitt des Basiselements ändert. Dadurch stellt die exakt definierte Schwenkbewegung des Steuerelements somit auch eine exakt definierte Auslenkbewegung des auszulenkenden biegbaren Körpers sicher.

In einer Variante kann das Steuerelement als ein Betätigungshebel aufgebaut sein, der am Schwenkabschnitt eine zum Basiskörper weisende Fußfläche besitzt, wobei der Kopfabschnitt des Basiselements eine zum Steuerelement weisende Stirnfläche besitzt, wobei zumindest die Fußfläche des Schwenkabschnitts des Steuerelements und/oder die Stirnfläche des Kopfabschnittes des Basiselements so gewölbt ist, dass die Fußfläche und die Stirnfläche aufeinander abrollbar sind, und wobei der Schwenkabschnitt des Steuerelements an seiner Außenseite den Anlenkpunkt des Bewegungsübertragungsdrahtkörpers hat und mit seiner Fußfläche auf der Stirnfläche des Kopfabschnittes des Basiselements schwenkbar sitzt. Dabei stehen sich die Fußfläche des Schwenkabschnitts des Steuerelements und die Stirnfläche des Kopfabschnittes des Basiselements gegenüber. Bei einer Schwenkbewegung des Steuerelements rollt die Fußfläche des Schwenkabschnitts des Steuerelements auf der Stirnfläche des Kopfabschnittes des Basiselements, wobei während dieser Abrollbewegung die Fußfläche und die Stirnfläche stets unter Punktkontakt zueinander stehen.

Beispielsweise kann die Fußfläche des Schwenkabschnitts des Steuerelements und/oder die Stirnfläche des Kopfabschnittes des Basiselements nach außen gewölbt sein.

In einer anderen Variante, die nicht unter den Schutzbereich des unabhängigen Anspruchs fällt, kann das Steuerelement als ein Betätigungshebel aufgebaut sein, der am Schwenkabschnitt in einen Hohlkugelabschnitt übergeht, der zumindest als Kugelringabschnitt ausgebildet ist, wobei der Kopfabschnitt des Basiselements kugelartig ausgebildet ist, und wobei der Hohlkugelabschnitt an seiner Außenseite den Anlenkpunkt des Bewegungsübertragungsdrahtkörpers hat und an seiner Innenkugelfläche auf dem kugelartigen Kopfabschnitt gleitfähig sitzt.

In dieser Variante bildet das Basiselement einen Innenkugelabschnitt, während das Steuerelement einen auf dem Innenkugelabschnitt sitzenden Hohlkugelabschnitt hat. Die Kugeldurchmesser des Innenkugelabschnittes und des Hohlkugelabschnittes sind so gewählt, dass eine Relativbewegung des Steuerelements zum Basiselement leicht möglich ist, aber eine exakt definierte Schwenkbewegung des Steuerelements zum Basiselement sichergestellt ist.

Dabei kann der Bewegungsübertragungsdrahtkörper äquatorial am Schwenkabschnitt des Steuerelements angelenkt sein.

Der Kopfabschnitt kann an der vom Steuerelement abgewandten Seite einstückig mit einem Stabelement als das Basiselement verbunden sein und das Stabelement kann in einem Stabelementhalter, von dem sich der Übertragungsführungskörper erstreckt, axial bewegbar sein, um den Bewegungsübertragungsdrahtkörper zu spannen.

Der Übertragungsführungskörper kann sich seitlich vom Stabelementhalter unter einem vorbestimmten Winkel von der Längsachse des Stabelements erstrecken.

Ein erstes Ende des Bewegungsübertragungsdrahtkörpers kann am Steuerelement angelenkt sein und ein entgegengesetztes zweites Ende des Bewegungsübertragungsdrahtkörpers kann am distalen Endabschnitt des auszulenkenden Körpers befestigt sein.

Der Übertragungsführungskörper kann einen Hohlraum besitzen, in dem der Bewegungsübertragungsdrahtkörper geführt wird.

Der Übertragungsführungskörper kann ein Schienenelement sein, an dem der Bewegungsübertragungsdrahtkörper geführt wird.

Das Steuerelement kann arretierbar sein, um eine Auslenkstellung des Steuerelements zu arretieren.

Das Steuerelement kann als Joystick vorgesehen sein, dessen Auslenkstellung durch eine Reibungsbremse arretierbar ist.

Ein, zwei, drei, vier oder mehr Bewegungsübertragungsdrahtkörper können vorgesehen sein, von denen die ersten Enden gleichmäßig zueinander beabstandet äquatorial am Steuerelement angelenkt sind und die entgegengesetzten zweiten Enden am distalen Endabschnitt des auszulenkenden Körpers in entsprechender Weise gleichmäßig zueinander beabstandet befestigt sind.

### Kurzbeschreibung der Zeichnungen

Figur 1 zeigt eine schematische Schnittansicht der Auslenkbewegungsübertragungseinrichtung eines ersten Ausführungsbeispiels im nicht ausgelenkten Zustand.
Figur 2 zeigt eine schematische Schnittansicht der Auslenkbewegungsübertragungseinrichtung des ersten Ausführungsbeispiels bei einer Auslenkung nach links.
Figur 3 zeigt eine schematische Schnittansicht der Auslenkbewegungsübertragungseinrichtung des ersten Ausführungsbeispiels bei einer Auslenkung nach rechts.
Figur 4 zeigt eine schematische perspektivische aufgeschnittene Darstellung gemäß Figur 1.
Figur 5 zeigt in einer schematischen perspektivischen ausschnittartigen Darstellung Einzelheiten der Anbindung der Drahtkörper am Steuerelement des ersten Ausführungsbeispiels und wie die Drahtkörper zum Katheterschlauch geführt werden.
Figur 6 zeigt eine schematische Schnittansicht der Auslenkbewegungsübertragungseinrichtung eines zweiten Ausführungsbeispiels im nicht ausgelenkten Zustand.
Figur 7 zeigt eine schematische Schnittansicht der Auslenkbewegungsübertragungseinrichtung des zweiten Ausführungsbeispiels bei einer Auslenkung nach links.
Figur 8 zeigt eine schematische Schnittansicht der Auslenkbewegungsübertragungseinrichtung des zweiten Ausführungsbeispiels bei einer Auslenkung nach rechts.
Figur 9 zeigt in einer schematischen perspektivischen ausschnittartigen Darstellung Einzelheiten der Anbindung der Drahtkörper am Steuerelement des zweiten Ausführungsbeispiels und wie die Drahtkörper zum Katheterschlauch geführt werden.

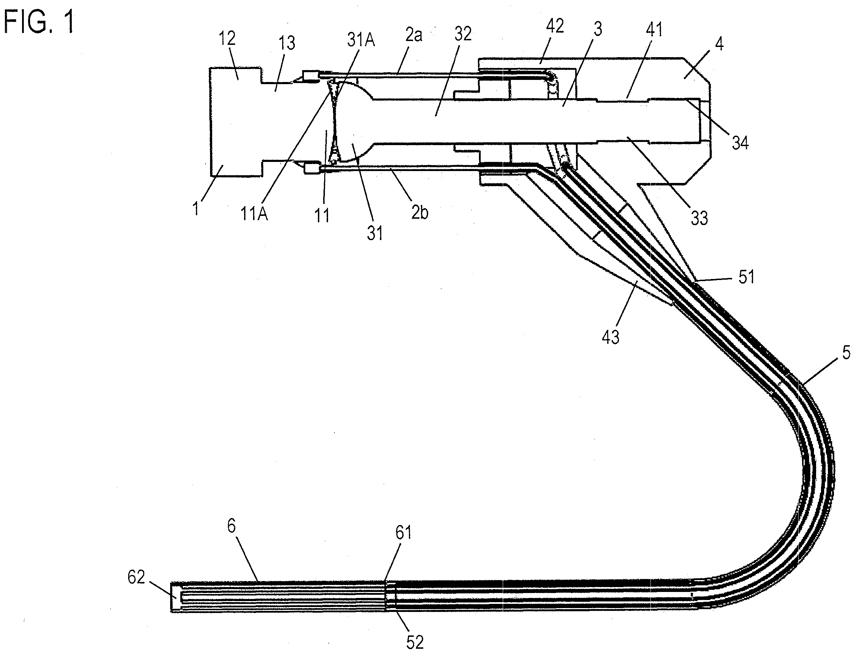

Nachstehend ist ein Ausführungsbeispiel der vorliegenden Erfindung detailliert anhand der Figuren 1 bis 5 beschrieben.

### Ausführungsbeispiel

Zunächst ist ein Ausführungsbeispiel der vorliegenden Erfindung detailliert anhand der Figuren 1 - 5 beschrieben.

Das Ausführungsbeispiel zeigt ein Auslenkbewegungsübertragungselement, welches bei einem Endoskop für eine Endoskopdeflectingsteuerung eingesetzt wird.

Diese Auslenkbewegungsübertragungseinrichtung besteht im vorliegenden Ausführungsbeispiel aus einem Steuerelement 1, mehreren Drahtkörpern 2, einem Stabelement 3 als Basiselement, einem Stabelementhalter 4, einem Katheterschlauch 5 und einem biegbaren Körper als Deflectingabschnitt 6.

Das Steuerelement 1 besteht aus einem zylinderartigen Element mit einem Steuerkopf 12, an dessen Unterseite ein Stababschnitt 13 zentrisch angeordnet ist. Der Stababschnitt 13 besitzt an dem zum Steuerkopf 12 entgegengesetzten Ende einen Fußabschnitt 11. Der Stababschnitt 13 hat einen gleichbleibenden Außendurchmesser. Der Fußabschnitt 11 hat einen Außendurchmesser, der in die zum Steuerkopf 12 entgegengesetzte Richtung zunimmt.

Am Fußabschnitt 11 schwenkt das Steuerelement 1 relativ zum Stabelement 3; daher ist der Fußabschnitt 11 des Steuerelements 1 als Schwenkabschnitt 11 bezeichnet.

Der Schwenkabschnitt 11 besitzt an der zum Steuerkopf 12 entgegengesetzten Seite eine als Fußfläche 11A ausgebildete Endfläche. Im vorliegenden Ausführungsbeispiel ist die Fußfläche 11A nach außen gewölbt. Anders ausgedrückt nimmt der in Längsrichtung des zylindrischen Steuerelements 1 gemessene Abstand zwischen der Fußfläche 11A zu der zum Fuß entgegengesetzten Stirnfläche des Steuerkopfes 12 vom Außenumfang zur Mitte hin zu. Die Fußfläche 11A bildet daher einen Abschnitt einer Kugelfläche mit einem vorbestimmten Radius, dessen Mittelpunkt auf der gedachten verlängerten Achse des Steuerelements 1 liegt.

Das Steuerelement 1 ist rotationssymmetrisch, wie dies in den Figuren 1 - 4 erkennbar ist. Das Steuerelement 1 ist aus einem Kunststoffmaterial hergestellt, kann aber auch aus Metall gefertigt sein.

Der Fußfläche 11A des Schwenkabschnittes 11 steht eine Stirnfläche 31A eines Kopfabschnittes 31 des Stabelementes 3 gegenüber, wie dies in den Figuren 1 - 4 dargestellt ist.

Das Stabelement 3 besitzt einen Längszylinder 32, der an seiner distalen Seite in den Kopfabschnitt 31 übergeht und an seinem distalen Endabschnitt ein Schraubende 34 aufweist, das im vorliegenden Ausführungsbeispiel als Innenvierkant ausgearbeitet ist. Distal von dem Vierkantende 34 besitzt das Stabelement 3 an seiner Außenzylinderfläche einen Außengewindeabschnitt 33. Das Stabelement 3 ist rotationssymmetrisch aufgebaut. Im Übrigen sind der Kopfabschnitt 31, der Längszylinder 32 und das Vierkantende 34 als ein einstückiges Stabelement aufgebaut. Der Längszylinder 32 des Stabelementes 3 ist mit Ausnahme des an ihn vorgesehenen Gewindeabschnittes 33 als ein Zylinder mit einer glatten Außenoberfläche ausgebildet.

Im vorliegenden Ausführungsbeispiel ist die Stirnfläche 31A ebenfalls nach außen zum Steuerelement 1 hin gewölbt. Anders ausgedrückt erhebt sich die Fußfläche 11A vom Außenumfang zur Mitte hin in Richtung zum Steuerelement 1. Die Stirnfläche 31A bildet dabei einen Abschnitt einer Kugelfläche mit einem vorbestimmten Radius, dessen Mittelpunkt auf der gedachten verlängerten Achse des Stabelements 3 liegt.

Die Fußfläche 11A und die Stirnfläche 31A stehen sich gegenüber und befinden sich in Kontakt zueinander. Somit sitzt der Schwenkabschnitt 11 des Steuerelements 1 mit seiner Fußfläche 11A schwenkbar auf der Stirnfläche 31A des Kopfabschnittes 31 des Stabelements 3.

Im nicht geschwenkten Zustand liegen der Schwenkabschnitt 11 des Steuerelements 1 und der Kopfabschnitt 31 des Stabelements 3 auf der gleichen Mittelachse, da im nicht geschwenkten Zustand das Steuerelement 1 und das Stabelement 3 koaxial zueinander angeordnet sind. Somit berühren sich im nicht geschwenkten Zustand der Mittelpunkt der Fußfläche 11A und der Mittelpunkt der Stirnfläche 31A. Wenn das Steuerelement 1 geschwenkt wird, also der Schwenkabschnitt 11 relativ zum Kopfabschnitt 31 des Stabelements 3 geneigt wird, rollt die Fußfläche 11A auf der Stirnfläche 31A.

Das Steuerelement 1 sitzt somit als ein Joystick auf dem Kopfabschnitt 31 des Stabelementes 3.

Wie dies in den Figuren 1 - 4 gezeigt ist, ist das Stabelement 3 in dem Stabelementhalter 4 angeordnet. Der Stabelementhalter 4 ist als ein Zylinderelement 42 gebildet, das rotationssymmetrisch aufgebaut ist. Das Zylinderelement 42 weist insbesondere an der zum Steuerelement 1 hin weisenden Seite einen Hohlraum und einen Boden an der vom Steuerelement 1 abgewandten Seite des Stabelementhalters 4 auf. Der Boden des Stabelementhalters 4 weist einen konzentrischen Innenkanal auf. In dem konzentrischen Innenkanal ist an einem Abschnitt von diesem ein Innengewinde 41 ausgearbeitet. Wie dies in den Figuren 1 - 4 schematisch angedeutet ist, sitzt das Außengewinde 33 des Stabelementes 3 an dem Innengewinde 41 des Stabelementhalters 4. Durch eine Schraubbewegung kann das Stabelement 3 relativ zum Stabelementhalter 4 konzentrisch hinein- oder herausgeschraubt werden. Zum Zwecke der Ausführung der Schraubbewegung wird ein entsprechendes Werkzeug in das Vierkantende 34 des Stabelementes 3 eingesetzt. Andere Relativbewegungstechniken sind möglich, wie dies unter "Alternativen" am Ende der Beschreibung erläutert ist.

An einem Abschnitt seiner Außenumfangsseite besitzt das Zylinderelement 42 des Stabelementhalters 4 ein Katheteranschlusselement 43. Im vorliegenden Ausführungsbeispiel erstreckt sich das Katheteranschlusselement 43 unter einem spitzen Winkel relativ zu dem Zylinderelement 42 des Stabelementes 4, wie dies aus den Zeichnungen hervorgeht.

Insbesondere ist das Katheteranschlusselement 43 als ein rundes Hohlprofil ausgearbeitet, das quasi eine Kanalabzweigung aus dem distalen Hohlraum des Zylinderelementes 42 darstellt. Das Katheteranschlusselement 43 ist zylinderartig ausgebildet, wobei es sich in Richtung vom Zylinderelement 42 weg verjüngt. Im Inneren besitzt das Katheteranschlusselement 43 einen konzentrischen Kanal, in welchem die Drahtkörper 2 geführt werden. An seinem distalen Ende besitzt das Katheteranschlusselement 43 eine kreisartige Mündung.

An der kreisartigen Mündung des Katheteranschlusselementes 43 ist der Katheterschlauch 5 befestigt. Insbesondere sitzt das proximale Ende 51 des Katheterschlauches 5 an der Mündung des Katheteranschlusselementes 43.

An seinem distalen Ende besitzt der Katheterschlauch einen in ihm aufgenommenen Ring 52. Der Ring 52 bildet das distale Ende des Katheterschlauches und den Übergang zu dem Deflectingabschnitt 6.

Der Deflectingabschnitt 6 ist ein biegbarer Körper, der in bekannter Weise aus einem elastischen Material hergestellt ist. An seinem proximalen Ende besitzt der Deflectingabschnitt einen Deflectinganschluss 61, an dem er an dem Ring 52 des Katheterschlauches 5 angeschlossen ist. An dem distalen Ende besitzt der Deflectingabschnitt eine Deflectingkappe 62, an der eine Kamera, ein Laser und/oder eine Kamera etc. angeordnet sind. Weitere Funktionseinheiten können an der Deflectingkappe 62 integriert werden.

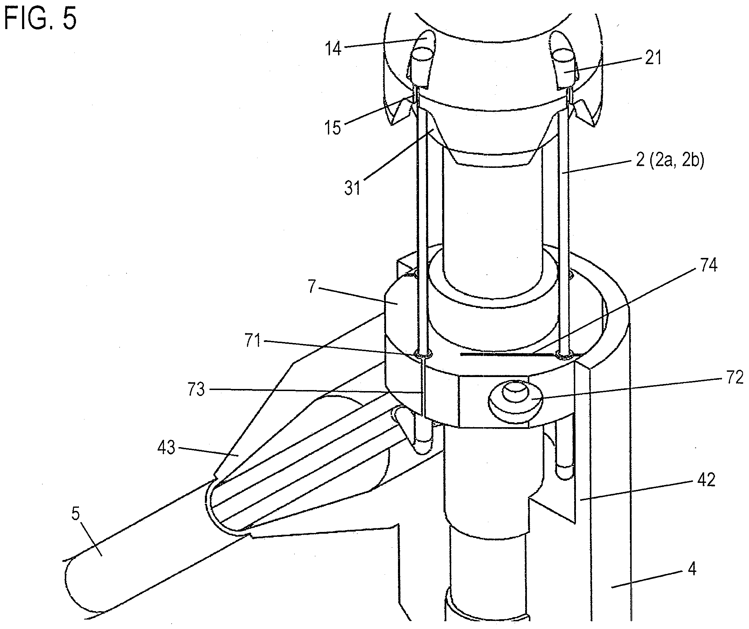

Wie dies in Figur 5 gezeigt ist, sind an der Außenumfangsfläche des Schwenkabschnittes 11 des Steuerelements 1 mehrere Einhängausbauchungen 14 vorgesehen. Im vorliegenden Ausführungsbeispiel sind vier Einhängausbauchungen 14 an der Außenumfangsfläche des Schwenkabschnittes 11 vorgesehen. Insbesondere sind die Einhängausbauchungen 14 an der Außenumfangsfläche des Schwenkabschnittes 11 eingeformte im Querschnitt kreisartige Vertiefungen mit einem Boden, wobei der Boden ungefähr senkrecht zu einer Erstreckungsrichtung der Einhängausbauchung 14 verläuft. Anders ausgedrückt erstreckt sich der Boden der Einhängausbauchung 14 ungefähr parallel zu der Stirnfläche des Steuerkopfes 12, die an der zu der Fußfläche 11A entgegengesetzten Seite des Steuerelements 1 ausgebildet ist.

Bei der Herstellung der Einhängausbauchung 14 kann der Schwenkabschnitt 11 so gefräßt werden, dass die Einhängausbauchung als ein seitlich offenes Sackloch an der Außenumfangsfläche des Schwenkabschnittes 11 entsteht. Beliebige andere Herstellverfahren sind hierbei denkbar. Der Außendurchmesser der Einhängausbauchung 14 ist derart gewählt, dass ein Tonnennippel 21 des Drahtkörpers 2 in die Einhängausbauchung 14 hineinpasst. Am Boden der Einhängausbauchung 14, das heißt an dem distalen Ende der Einhängausbauchung 14 ist ein Kanal 15 als Drahtkörpereinhängung ausgearbeitet, der sich koaxial zu der Längserstreckung des Steuerelementes 1 erstreckt und einen Durchmesser hat, der größer als der Außendurchmesser des Drahtkörpers 2 ist, aber kleiner als der Außendurchmesser des Tonnennippels 21 des Drahtkörpers 2 ist. Anders ausgedrückt sind die Einhängausbauchung 14 und die Drahtkörpereinhängung 15 ähnlich wie Baudenzugeinhängungen am Fahrrad so vorgesehen, dass ein Tonnennippel 21 eines Drahtkörpers 2 darin eingehängt werden kann. Das Tonnennippel bildet im eingehängten Zustand des Drahtkörpers 2 das proximale Ende des Drahtkörpers 2.

Im vorliegenden Ausführungsbeispiel sind vier Drahtkörper 2 vorgesehen, von denen in den Figuren 1 bis 4 jeweils zwei Drahtkörper, das heißt der Drahtkörper 2a und der Drahtkörper 2b, dargestellt sind. Die Anzahl an Drahtkörpern 2 ist hierbei nicht begrenzt. Es kann auch lediglich ein einziger Drahtkörper 2 vorgesehen sein. Es können zwei, drei, vier oder mehr Drahtkörper vorgesehen sein. Sollten zwei oder mehr Drahtkörper 2 vorgesehen sein, sind die entsprechenden Einhängausbauchungen 14 gleichmäßig zueinander beabstandet an der Außenumfangsfläche des Schwenkabschnittes 11 angeordnet.

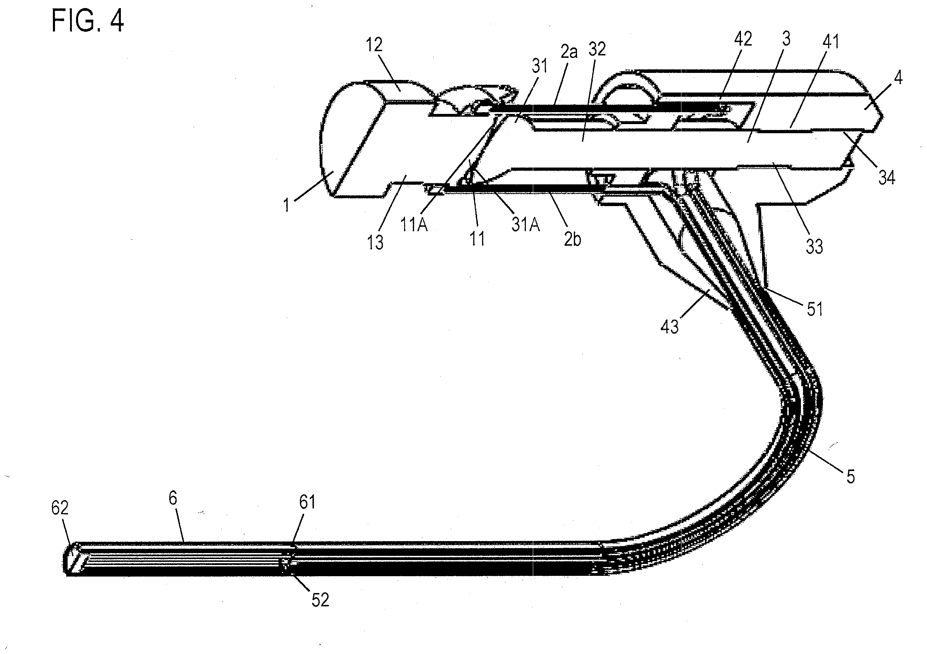

Wie dies in Figur 4 gezeigt ist, besitzt das Zylinderelement 42 an seinem proximalen Ende das heißt an seinem zum Steuerelement 1 weisenden Ende eine Zugangsöffnung zu einem Hohlraum. In diese Zugangsöffnung ist ein Drahtkörperführungsring 7 so eingesetzt, dass die proximale Fläche, das heißt die zum Steuerelement 1 weisende Fläche des Drahtführungsringes 7 zu der proximalen, das heißt zum Steuerelement 1 weisenden Stirnfläche des Zylinderelementes 42 fluchtet. Der Drahtführungsring 7 ist mit tangentialen Schlitzen 74 in gleicher Anzahl, wie Drahtführungen 2 vorhanden sind, versehen, wie dies in Figur 5 gezeigt ist. In den Schlitzen 74 sind Drahtführungsöffnungen 71 gebohrt, die koaxial zur Mittelachse des Drahtführungsrings 7 verlaufen. Im übrigen ist die Mittelachse des Drahtführungsrings 7 koaxial zu den jeweiligen Achsen des Stabelementes 3 und des Zylinderelementes 42 des Stabelementhalters 4. Genauer gesagt ist der Abstand jeder Einhängausbauchung 14 zu der Mittelachse des Steuerelementes 1 genauso groß wie der radiale Abstand zwischen der Drahtführungsbohrung 71 und der Mittelachse des Drahtführungsringes 7. Jede Drahtführungsbohrung 71 ist zu einer zugehörigen Drahtkörpereinhängung 15 ausgemittelt.

Das Schlitzende 73 jedes tangentialen Schlitzes 74 befindet sich an der Außenumfangsfläche des flachen Drahtführungsringes 7, wie dies in Figur 5 gezeigt ist. An der Außenumfangsfläche des flachen Drahtführungsringes 7 ist außerdem eine Gewindebohrung 72 vorgesehen, die eine Befestigungsschraube aufnimmt, mit der der Drahtführungsring 7 am Zylinderelement 42 des Stabelementhalters 4 fixiert wird. Obwohl in Figur 5 nur eine Gewindebohrung 72 für eine Befestigungsschraube gezeigt ist, können auch mehrere Gewindebohrungen 72 für entsprechend mehrere Befestigungsschrauben am Drahtführungsring 7 vorgesehen werden.

Die Drahtkörper 2 sind durch den Katheterschlauch 5 und durch den Ring 52 des Katheterschlauches hindurchgeführt und sind an der Deflectingkappe 62 des Deflectingabschnittes 6 verankert. Insbesondere sind die Drahtkörper 2 so an der Deflectingkappe 62 verankert, dass sie gleichmäßig zueinander beabstandet und in der gleichen Reihenfolge wie am Schwenkabschnitt 11 angeordnet sind.

Der Ring 52 weist Öffnungen für die Drahtkörper 2 in entsprechender Weise wie bei der Ausgestaltung des Drahtführungsringes 7 auf.

Die Länge jedes Drahtkörpers 2 vom Befestigungspunkt an der Deflectingkappe 62 bis zum Befestigungspunkt am Schwenkabschnitt 11 ist stets gleich.

### Funktionsweise

Das Steuerelement 1 kann wie ein Joystick betätigt werden, wobei sein Schwenkabschnitt 11 auf dem Kopfabschnitt 31 des Stabelements 3 rollend bewegt werden kann. Wenn somit der Schwenkabschnitt 11 relativ zum Kopfabschnitt 31 des Stabelements 3 geneigt wird, rollt die Fußfläche 11A auf der Stirnfläche 31A. Dadurch werden die am Schwenkabschnitt 11 angeordneten Enden der Drahtkörper 2 relativ zu der Baugruppe aus Stabelement 3, Stabelementhalter 4 und Katheterschlauch 5 gezogen oder gedrückt.

Ein Schwenkvorgang des Joysticks 1 relativ zum Stabelement 3 in beliebiger Richtung ist dadurch möglich. Die Richtung und das Ausmaß der Auslenkbewegung des Joysticks 1 relativ zum Stabelement 3 wird dann durch die an der Deflectingkappe 62 angeordneten Drahtkörper 2 auf den als biegbaren Körper ausgestalteten Deflectingabschnitt 6 übertragen.

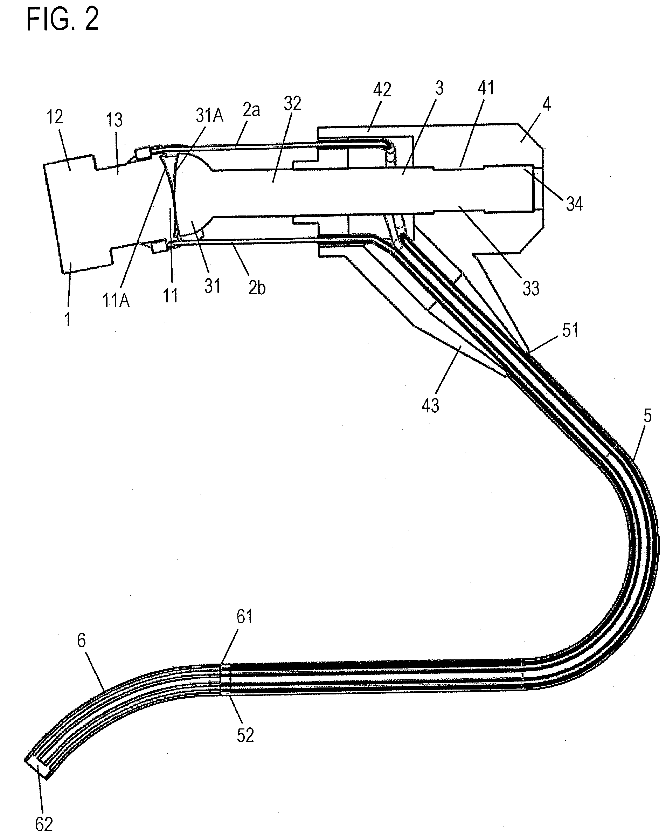

Anders ausgedrückt, wenn, wie dies in Figur 2 gezeigt ist, der Joystick 1 relativ zum Stabelement 3 nach links bewegt wird, wird der rechte Drahtkörper 2a gezogen, womit im Deflectingabschnitt 6 der rechte Drahtkörper 2a die Deflectingkappe 62 in proximaler Richtung zieht. Gleichzeitig wird der linke Drahtkörper 2b gedrückt, womit im Deflectingabschnitt 6 der linke Drahtkörper 2b die Deflectingkappe 62 in distaler Richtung drückt. Somit vollführt in Figur 2 der Deflectingabschnitt eine nach links gerichtete Bewegung.

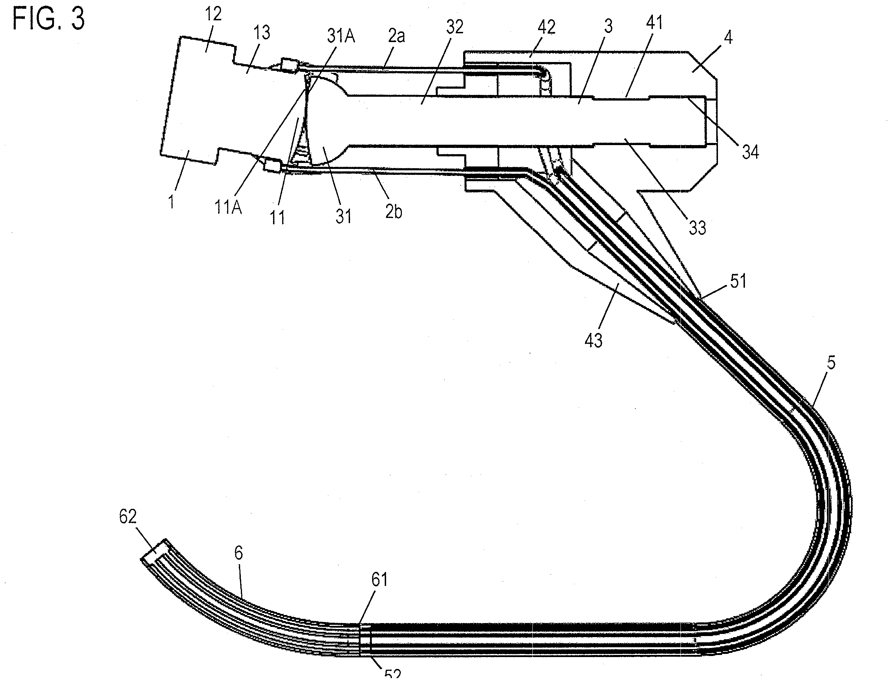

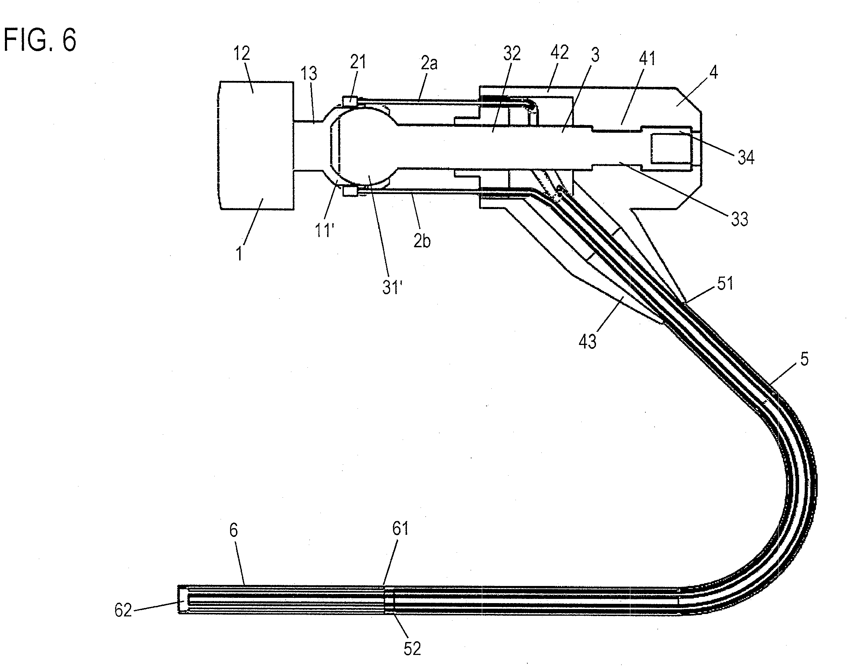

Wenn der Joystick 1 relativ zum Stabelement 3 nach rechts bewegt wird, vollführt der Deflectingabschnitt eine nach rechts gerichtete Bewegung, wie dies in Figur 3 der Fall ist. Weiteres Beispiel hilfreich zum Verständnis der Erfindung Nachstehend ist ein Beispiel detailliert anhand der Figuren 6 - 9 beschrieben, das nicht unter den Schutzbereich des Anspruchs 1 fällt, jedoch hilfreich ist für das Verständnis der Erfindung. Die Auslenkbewegungsübertragungseinrichtung besteht auch im vorliegenden Ausführungsbeispiel aus einem Steuerelement 1, mehreren Drahtkörpern 2, einem Stabelement 3, einem Stabelementhalter 4, einem Katheterschlauch 5 und einem biegbaren Körper als Deflectingabschnitt 6.

Das Steuerelement 1 besteht aus einem zylindrischen Element mit einem Steuerkopf 12, an dessen Unterseite ein Stababschnitt 13 zentrisch angeordnet ist, der aber anders als im vorherigen Ausführungsbeispiel in einen Hohlkugelabschnitt 11' übergeht, an dessen Außenfläche die Drahtkörper 2 verankert sind. Der Hohlkugelabschnitt 11' ist an seiner zum Kopf 12 abgewandten Seite hin offen. Insbesondere ist beispielsweise die Öffnung am Hohlkugelabschnitt 11' derart, dass der Hohlkugelabschnitt 11' zirka 9/10 einer Kugel ausmacht von der zirka 1/10 abgeschnitten ist.

Das Steuerelement 1 ist rotationssymmetrisch, wie dies in den Figuren 6 - 8 gezeigt ist. Das Steuerelement 1 ist aus einem Kunststoffmaterial hergestellt, kann aber auch aus Metall gefertigt sein.

Das Steuerelement 1 sitzt als ein Joystick auf einem Kopf 31 des Stabelementes 3. Insbesondere sitzt der Hohlkugelabschnitt 11' des Steuerelementes 1 auf einem Gegenkugelabschnitt 31', der in diesem Ausführungsbeispiel den Kopf des Stabelementes 3 bildet. Der Gegenkugelabschnitt 31' ist dabei derart ausgebildet, dass er eine Kugelform in einer derartigen Größe hat, dass der darauf sitzende Hohlkugelabschnitt 11' leichtgängig bewegbar ist. Die Maßbeziehung zwischen dem Gegenkugelabschnitt 31' und dem Hohlkugelabschnitt 11' sind dabei derart, dass eine Relativbewegung des Steuerelementes 1 zum Stabelement 3 ohne große Kraftanstrengung des Anwenders möglich ist, andererseits aber der Hohlkugelabschnitt 11' nicht lose auf dem Gegenkugelabschnitt 31' sitzt.

Das Stabelement 3 besitzt einen Längszylinder 32, der an seiner distalen Seite in den Gegenkugelabschnitt 31' übergeht und an seinem distalen Endabschnitt ein Schraubende 34 aufweist, das zum Beispiel als Innenvierkant ausgearbeitet ist. Distal von dem Vierkantende 34 besitzt das Stabelement 3 an seiner Außenzylinderfläche einen Außengewindeabschnitt 33. Das Stabelement 3 ist rotationssymmetrisch aufgebaut. Der Gegenkugelabschnitt 31', der Längszylinder 32 und das Vierkantende 34 sind als ein einstückiges Stabelement aufgebaut. Der Längszylinder 32 des Stabelementes 3 ist mit Ausnahme des an ihn vorgesehenen Gewindeabschnittes 33 als ein Zylinder mit einer glatten Außenoberfläche ausgebildet.

Wie dies in den Figuren 6 - 8 gezeigt ist, sitzt das Stabelement 3 in einem Stabelementhalter 4. Der Stabelementhalter 4 besteht wie im ersten Ausführungsbeispiel aus einem Zylinderelement 42, das rotationssymmetrisch aufgebaut ist und einen zentrischen Innenkanal aufweist. Das Zylinderelement 42 weist den zum Steuerelement 1 hin weisenden Hohlraum und einen Boden an der vom Steuerelement 1 abgewandten Seite des Stabelementhalters 4 auf. Der Boden des Stabelementhalters 4 weist den konzentrischen Innenkanal auf. In dem konzentrischen Innenkanal ist ein Innengewinde 41 vorgesehen. Wie dies in den Figuren schematisch angedeutet ist, sitzt das Außengewinde 33 des Stabelementes 3 an dem Innengewinde 41 des Stabelementhalters 4, wobei durch eine Schraubbewegung das Stabelement 3 relativ zum Stabelementhalter 4 konzentrisch hinein oder herausgeschraubt werden kann. Zum Zwecke der Ausführung der Schraubbewegung wird ein entsprechendes Werkzeug in das Vierkantende 34 des Stabelementes 3 eingesetzt.

An seiner Außenumfangsseite besitzt das Zylinderelement 42 des Stabelementhalters 4 ein Katheteranschlusselement 43. Auch im vorliegenden Ausführungsbeispiel erstreckt sich das Katheteranschlusselement 43 unter einem spitzen Winkel relativ zu dem Zylinderelement 42 des Stabelementes 4, wie dies aus den Zeichnungen hervorgeht.

Das Katheteranschlusselement 43 ist als ein rundes Hohlprofil ausgearbeitet, das eine Kanalabzweigung aus dem distalen Hohlraum des Zylinderelementes 42 bildet. Das Katheteranschlusselement 43 ist zylinderartig ausgebildet und verjüngt sich in vom Zylinderelement 42 weg weisender Richtung. Im Inneren besitzt das Katheteranschlusselement 43 einen konzentrischen Kanal zur Führung der Drahtkörper 2. An seinem distalen Ende hat das Katheteranschlusselement 43 eine kreisartige Mündung.

An der kreisartigen Mündung des Katheteranschlusselementes 43 ist der Katheterschlauch 5 befestigt. Insbesondere sitzt das proximale Ende 51 des Katheterschlauches 5 an der Mündung des Katheteranschlusselementes 43. An seinem distalen Ende besitzt der Katheterschlauch einen in ihm aufgenommenen Ring 52. Der Ring 52 bildet das distale Ende des Katheterschlauches und den Übergang zu dem Deflectingabschnitt 6.

Der Deflectingabschnitt 6 ist ein biegbarer Körper, der in bekannter Weise aus einem elastischen Material hergestellt ist. An seinem proximalen Ende besitzt der Deflectingabschnitt einen Deflectinganschluss 61, an dem er an dem Ring 52 des Katheterschlauches 5 angeschlossen ist. An dem distalen Ende besitzt der Deflectingabschnitt eine Deflectingkappe 62, an der eine Kamera, ein Laser und/oder eine Kamera etc. angeordnet sind. Weitere Funktionseinheiten können an der Deflectingkappe 62 integriert werden.

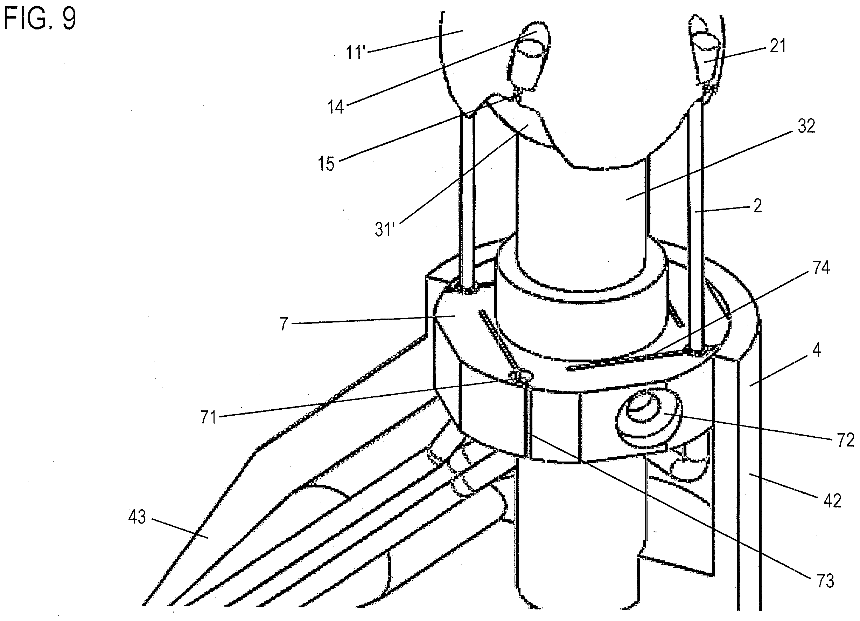

Figur 9 zeigt in einer schematischen perspektivischen ausschnittartigen Darstellung Einzelheiten der Anbindung der Drahtkörper am Steuerelement des zweiten Ausführungsbeispiels und wie die Drahtkörper zum Katheterschlauch geführt werden. In Figur ist der besseren Übersichtlichkeit wegen der vordere linke Drahtkörper 2 nicht dargestellt.

Wie dies in Figur 9 gezeigt ist, sind an der Außenumfangsfläche des Hohlkugelabschnittes 11' an der Äquatorlinie des Hohlkugelabschnittes 11' mehrere Einhängausbauchungen 14 vorgesehen. Im vorliegenden Ausführungsbeispiel sind vier Einhängausbauchungen 14 am Äquator des Hohlkugelabschnittes 11' vorgesehen. Insbesondere sind die Einhängausbauchungen 14 am Hohlkugelabschnitt 11' eingeformte im Querschnitt kreisartige Vertiefungen mit einem Boden, wobei der Boden ungefähr senkrecht zu einer Erstreckungsrichtung der Einhängausbauchung 14 verläuft und sich an der Äquatorlinie genauer gesagt senkrecht zum Äquator des Hohlkugelabschnittes 11' befinden. Bei der Herstellung der Einhängausbauchung 14 kann der Hohlkugelabschnitt 11' von der proximalen Seite aus z.B. durch Fräsen hergestellt werden, sodass die Einhängausbauchung als ein seitlich offenes Sackloch an der Außenumfangsfläche des Hohlkugelabschnittes 11' entsteht. Beliebige andere Herstellverfahren sind hierbei denkbar. Der Außendurchmesser der Einhängausbauchung 14 ist derart gewählt, dass ein Tonnennippel 21 des Drahtkörpers 2 in die Einhängausbauchung 14 hineinpasst. Am Boden der Einhängausbauchung 14, das heißt an dem distalen Ende der Einhängausbauchung 14 ist ein Kanal 15 als Drahtkörpereinhängung ausgearbeitet, der sich koaxial zu der Längserstreckung des Steuerelementes 1 erstreckt und einen Durchmesser hat, der größer als der Außendurchmesser des Drahtkörpers 2 ist, aber kleiner als der Außendurchmesser des Tonnennippels 21 des Drahtes 2 ist. Anders ausgedrückt sind die Einhängausbauchung 14 und die Drahtkörpereinhängung 15 ähnlich wie im ersten Ausführungsbeispiel.

Die Anzahl an Drahtkörpern 2 ist hierbei ebenfalls nicht begrenzt. Sollten zwei oder mehr Drahtkörper 2 vorgesehen sein, sind die entsprechenden Einhängausbauchungen 14 gleichmäßig zueinander beabstandet am Äquator des Hohlkugelabschnittes 11' angeordnet.

Ein Drahtkörperführungsring 7 mit tangentialen Schlitzen 74 in gleicher Anzahl, wie Drahtführungen 2 vorhanden sind, ist wie im ersten Ausführungsbeispiel vorgesehen, wie dies in Figur 9 gezeigt ist. In den Schlitzen 74 sind Drahtführungsöffnungen 71 gebohrt, die koaxial zu den gemeinsamen Achsen des Stabelementes 3 und des Zylinderelementes 42 des Stabelementhalters 4 verlaufen.

Das Schlitzende 73 jedes tangentialen Schlitzes 74 befindet sich an der Außenumfangsfläche des flachen Drahtführungsringes 7, wie dies in Figur 9 gezeigt ist. An der Außenumfangsfläche des flachen Drahtführungsringes 7 ist wie im ersten Ausführungsbeispiel eine Gewindebohrung 72 vorgesehen, die eine Befestigungsschraube aufnimmt, mit der der Drahtführungsring 7 am Zylinderelement 42 des Stabelementhalters 4 fixiert wird. Obwohl in Figur 9 nur eine Gewindebohrung 72 für eine Befestigungsschraube gezeigt ist, können auch mehrere eine Gewindebohrungen 72 für entsprechend mehrere Befestigungsschrauben am Drahtführungsring 7 vorgesehen werden.

Die Drahtkörper 2 sind auch hier durch den Katheterschlauch 5 und durch den Ring 52 des Katheterschlauches hindurchgeführt und sind an der Deflectingkappe 62 des Deflectingabschnittes 6 verankert. Die Drahtkörper 2 sind so an der Deflectingkappe 62 verankert, dass sie gleichmäßig zueinander beabstandet und in der gleichen Reihenfolge wie am Hohlkugelabschnitt 11' befestigt sind. Der Ring 52 weist Öffnungen für die Drahtkörper 2 in entsprechender Weise wie bei der Ausgestaltung des Drahtführungsringes 7 auf.

### Funktionsweise

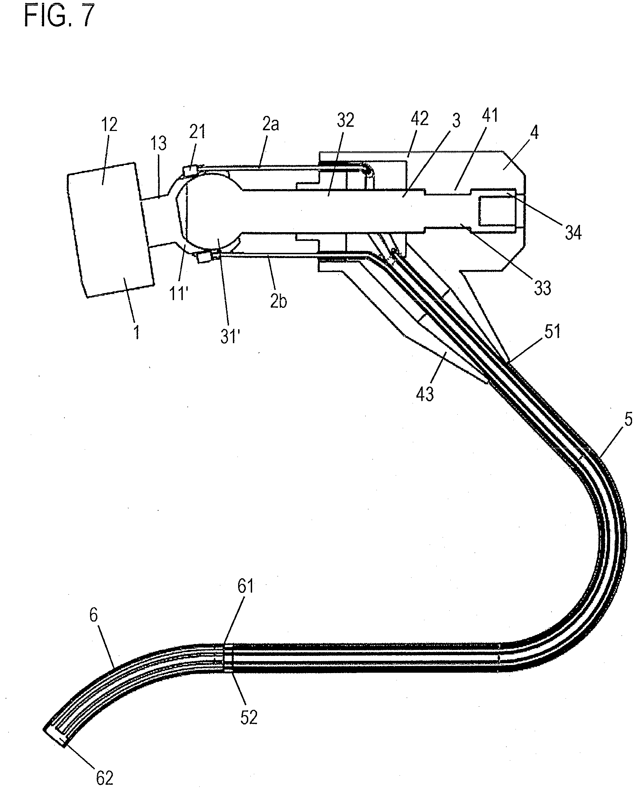

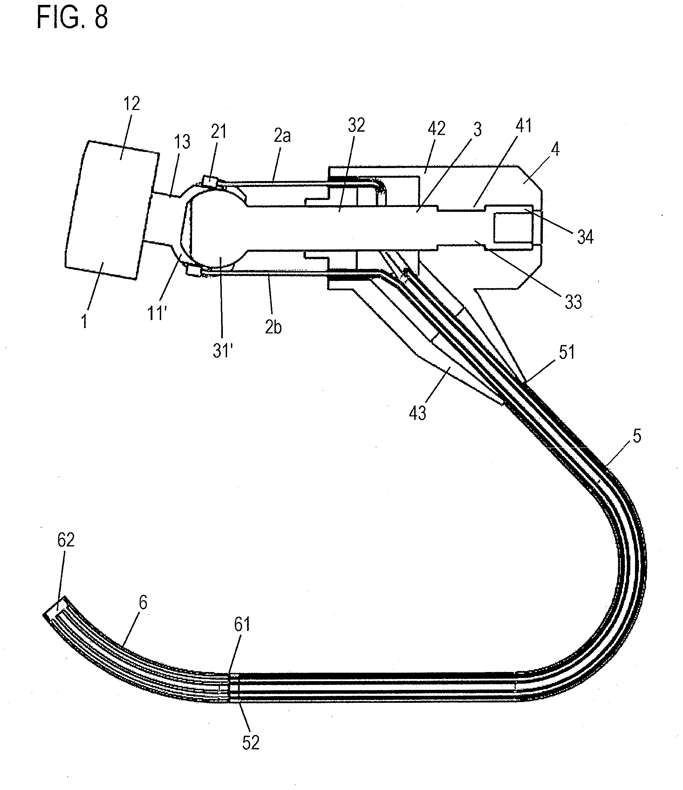

Das Steuerelement 1 kann wie im ersten Ausführungsbeispiel als ein Joystick betätigt werden. In diesem Ausführungsbeispiel kann dabei der Hohlkugelabschnitt 11' auf dem Gegenkugelabschnitt 31' des Stabelementes 3 bewegt werden. Ein Schwenkvorgang des Joysticks 1 relativ zum Stabelement 3 in beliebiger Richtung ist dadurch möglich. Die Richtung und das Ausmaß der Auslenkbewegung des Joysticks 1 relativ zum Stabelement 3 wird dann durch die an der Deflectingkappe 62 angeordneten Drahtkörper 2 auf den als biegbaren Körper ausgestalteten Deflectingabschnitt 6 übertragen. Anders ausgedrückt, wenn der Joystick 1 relativ zum Stabelement 3 nach links bewegt wird, vollführt der Deflectingabschnitt eine nach links gerichtete Bewegung, wie dies in Figur 7 gezeigt ist. Wenn der Joystick 1 relativ zum Stabelement 3 nach rechts bewegt wird, vollführt der Deflectingabschnitt eine nach rechts gerichtete Bewegung, wie dies in Figur 8 gezeigt ist.

### Alternativen

Der Drahtkörper 2 ist in der in Figur 4 gezeigten Einhängausbauchung 14 in der Form eines Tonnennippels 21 eingehängt. Diese Ausführung, die nicht unter den Schutzbereich des unabhängigen Anspruchs fällt, ist nicht auf ein Tonnennippel beschränkt und das Nippel 21 kann als ein bekanntes Birnennippel ausgeführt sein, beliebige ähnliche Nippel können angewendet werden. Die Form der Einhängausbauchung 14 kann an die gewählte Nippelform angepasst werden.

Im ersten Ausführungsbeispiel ist die Fußfläche 11A nach außen gewölbt. Außerdem ist Stirnfläche 31A des Kopfabschnittes 31 nach außen gewölbt. Die Erfindung ist nicht darauf beschränkt. Die Auslenkbewegungsübertragungseinrichtung kann unter Ausnutzung des Prinzips der Erfindung auch so aufgebaut sein, dass die Fußfläche 11A eben gestaltet ist und die Stirnfläche 31A nach außen gewölbt ist. Andererseits kann die Auslenkbewegungsübertragungseinrichtung auch so aufgebaut sein, dass die Fußfläche 11A nach außen gewölbt ist und die Stirnfläche 31A eben gestaltet ist. Es ist auch eine Konstruktion denkbar, bei der die Stirnfläche 31A nach innen gewölbt ist und die Fußfläche 11A nach außen gewölbt ist, sofern der Krümmungsradius der Stirnfläche 31A größer als der Krümmungsradius der Fußfläche 11A ist. In ähnlicher Weise kann die Stirnfläche 31A nach außen gewölbt sein und die Fußfläche 11A nach innen gewölbt sein, sofern der Krümmungsradius der Stirnfläche 31A kleiner als der Krümmungsradius der Fußfläche 11A ist. Es ist lediglich ausreichend, dass die Fußfläche 11A sicher und kontrolliert auf der Stirnfläche 31A abrollen kann.

Im Beispiel hilfreich zum Verständnis der Erfindung ist die Größe des Hohlkugelabschnittes 11' so gewählt worden, dass sie 9/10 einer Kugel beträgt. Eine beliebige Hohlkugelformgröße des Hohlkugelabschnittes 11' kann gewählt werden, solange diese noch an dem Gegenkugelabschnitt 31' die Schwenkbewegung ausführen kann. Der Hohlkugelabschnitt 11' kann auch eine Hohlkugelringabschnittform haben, die sich um ein vorbestimmtes Mindestmaß parallel zur Achsrichtung des Steuerelements 1 zu beiden Seiten von der Äquatorlinie erstreckt und quasi ein Äquatorband bildet.

Das Vierkantende 34 dient der Ermöglichung einer Schraubbewegung, um an den Gewindeabschnitten 33 und 41 eine Relativbewegung des Stabelementes 3 zum Stabelementhalter 4 auszuführen. Dieses Beispiel ist nicht auf die Vierkantform am Ende 34 des Stabelementes 3 beschränkt. Eine Dreikantform, Achtkantform, andere polygonale Form kann gewählt werden. Im Prinzip kann jede beliebige Form, die das Angreifen eines die Drehbewegung des Stabelementes am Ende 34 erzeugenden Momentes ermöglicht, gewählt werden.

Im den Ausführungsbeispielen wird die Relativbewegung des Stabelementes 3 zum Stabelementhalter 4 durch die Gewindeabschnitte 33 und 41 bewerkstelligt. Durch die Bewegung des Stabelementes 3 relativ zum Stabelementhalter 4 werden die Drahtkörper 2 gespannt. Eine beliebige andere Bewegungsart des Stabelementes 3 zum Stabelementhalter 4 kann für diesen Zweck gewählt werden. Beispielsweise kann der Stabelementhalter 4 ein durchgehendes Innenzylinderloch aufweisen und das Stabelement 3 einen durchgehend zylindrischen Längszylinder 32 haben, wobei am Ende 34 des Stabelementes 3 eine Zugeinrichtung angebracht ist. Im Stabelementhalter 4 kann eine Gewindebohrung senkrecht zur Achse des Stabelementhalters vorgesehen werden, in der eine Feststellschraube sitzt, die den Längszylinder 32 in beliebiger Stellung relativ zum Stabelementhalter 4 arretieren kann.

In den Ausführungsbeispielen erstreckt sich das Katheteranschlusselement 43 unter einem spitzen Winkel unter Betrachtung von Figur 1 zum Stabelementhalter 4. Ein beliebiger Erstreckungswinkel des Katheteranschlusselementes 43 zum Stabelementhalter 4 kann gewählt werden.

In den Ausführungsbeispielen ist der Katheterschlauch 5 ein Übertragungsführungskörper, der einen Hohlraum besitzt, in dem der Bewegungsübertragungsdrahtkörper geführt wird. Bei einer Schwenkbewegung des Steuerelementes sind die Bewegungsübertragungsdrahtkörper 2 Zugkräften und Druckkräften ausgesetzt. Wenn diese Zugkräfte und Druckkräfte auf sie ausgeübt werden, müssen die Bewegungsübertragungsdrahtkörper 2 am Übertragungsführungskörper gleiten können. Der Übertragungsführungskörper kann hierbei einen geschlossenen Querschnitt haben, wie dies bei einem Katheterschlauch 5 der Fall ist. Der Übertragungsführungskörper kann ein Schienenelement oder Kastenelement sein, an dem der Bewegungsübertragungsdrahtkörper geführt wird. Der Querschnitt des Übertragungsführungskörpers kann an der Seite offen sein, an der die Bewegungsübertragungsdrahtkörper 2 nicht gleiten.

Das Steuerelement 1 kann arretierbar sein, um eine Auslenkstellung des Steuerelements 1 zu arretieren. Im zweiten Ausführungsbeispiel kann die Arretierung durch eine Feststellschraube erfolgen, die z.B. den Hohlkugelabschnitt 11' durchdringt und an der Oberfläche des Gegenkugelabschnittes 31' angreift und somit als eine Reibungsbremse so wirkt, dass eine bestimmte Auslenkstellung des Steuerelements 1 also des Steuerhebels per Reibungsbremse arretierbar ist. Darüber hinaus kann in allen Ausführungsbeispielen eine Arretierung erfolgen, indem der/die Bewegungsübertragungsdrahtkörper 2 z.B. am Drahtführungsring 7 oder am Zylinderelement 42 des Stabelementhalters 4 geklemmt werden. Sollten alle Drähte 2 z.B. durch eine am Drahtführungsring 7 oder am Zylinderelement 42 angebrachte Feststellklemme arretiert werden, wird dadurch eine sichere Arretierung einer Auslenkstellung des Steuerelements 1 erreicht. Andere technische Möglichkeiten zum Blockieren der Drähte 2 können gewählt werden. In den Ausführungsbeispielen ist die Auslenkbewegungsübertragungseinrichtung auf eine Endoskopdeflectingsteuerung bei einem Endoskop angewandt. Die Auslenkbewegungsübertragungseinrichtung kann auch auf anderen technischen Gebieten Anwendung finden. Ein Einsatz in Wasser führenden Kanälen, in Bergbaustollen etc. ist denkbar.

### Bezugszeichenliste

1 Steuerelement; Joystick
2, 2a, 2b Drahtkörper
3 Basiselement; Stabelement
4 Stabelementhalter
5 Katheterschlauch
6 biegbarer Körper, Deflectingabschnitt
7 Drahtführungsring
11 Schwenkabschnitt
11A Fußfläche des Schwenkabschnitts 11
11' Hohlkugelabschnitt
12 Kopf des Steuerelementes 1
13 Stababschnitt
14 Einhängausbauchung
15 Drahtkörpereinhängung
21 Tonnennippel
31 Kopfabschnitt
31A Stirnfläche des Kopfabschnittes 31
31' Gegenkugelabschnitt
32 Längszylinder
33 Gewindeabschnitt des Stabelementes 3
34 Vierkantende; distales Ende des Stabelementes 3
41 Gewindeabschnitt des Stabelementhalters 4
42 Zylinderelement
43 Katheteranschlusselement
51 Katheterschlauchanschluss
52 Ring
61 Deflectinganschluss
62 Deflectingkappe
71 Drahtführungsbohrung
72 Gewindebohrung für Befestigungsschraube
73 Schlitzeingang
74 tangentialer Schlitz

## Patentansprüche

1. Auslenkbewegungsübertragungseinrichtung mit
einem Steuerelement (1) zur Bewerkstelligung einer Auslenkbewegung, wobei das Steuerelement (1) einen Schwenkabschnitt (11; 11') besitzt, der an einem Kopfabschnitt (31; 31') eines Basiselements (3) abgestützt ist und zur Bewerkstelligung einer Auslenkbewegung relativ zum Kopfabschnitt (31; 31') des Basiselements (3) schwenkbar ist,
zumindest einem Bewegungsübertragungsdrahtkörper (2, 2a, 2b), der am Schwenkabschnitt (11; 11') des Steuerelements (1) angelenkt ist,
einem länglichen Übertragungsführungskörper (5), in dessen Längsrichtung der Bewegungsübertragungsdrahtkörper (2, 2a, 2b) geführt ist, und
einem auszulenkenden biegbaren Körper (6), der am vom Steuerelement (1) entgegengesetzten Ende des Übertragungsführungskörpers (5) sitzt und an dem der Bewegungsübertragungsdrahtkörper (2, 2a, 2b) beabstandet von der Verbindung (61) zum Übertragungsführungskörper (5) angebracht ist,
wobei das Steuerelement (1) als ein Betätigungshebel aufgebaut ist, der am Schwenkabschnitt (11) eine zum Basiskörper (3) weisende Fußfläche (11A) besitzt,
wobei der Kopfabschnitt (31) des Basiselements (3) eine zum Steuerelement (1) weisende Stirnfläche (31A) besitzt,
wobei der Schwenkabschnitt (11) des Steuerelements (1) an seiner Aussenseite den Anlenkpunkt (14) des Bewegungsübertragungsdrahtkörpers (2,2a,2b) hat, **dadurch gekennzeichnet, dass** die Fußfläche (11A) des Schwenkabschnitts (11) des Steuerelements (1) und die Stirnfläche (31A) des Kopfabschnittes (31) des Basiselements (3) jeweils zueinander hin so gewölbt sind, dass die Fußfläche (11A) und die Stirnfläche (31A) aufeinander abrollbar.

2. Auslenkbewegungsübertragungseinrichtung gemäß Anspruch 1, wobei der Kopfabschnitt (31; 31') an der vom Steuerelement (1) abgewandten Seite einstückig mit einem Stabelement (3) als das Basiselement verbunden ist und das Stabelement (3) in einem Stabelementhalter (4), von dem sich der Übertragungsführungskörper (5) erstreckt, axial bewegbar ist, um den Bewegungsübertragungsdrahtkörper (2, 2a, 2b) zu spannen.

3. Auslenkbewegungsübertragungseinrichtung gemäß Anspruch 2, wobei sich der Übertragungsführungskörper (5) seitlich vom Stabelementhalter (4) unter einem vorbestimmten Winkel von der Längsachse des Stabelements (3) erstreckt.

4. Auslenkbewegungsübertragungseinrichtung gemäß einem der Ansprüche 1 bis 3, wobei ein erstes Ende des Bewegungsübertragungsdrahtkörpers (2, 2a, 2b) am Steuerelement (1) angelenkt ist und ein entgegengesetztes zweites Ende des Bewegungsübertragungsdrahtkörpers (2, 2a, 2b) am distalen Endabschnitt des auszulenkenden Körpers (6) befestigt ist.

5. Auslenkbewegungsübertragungseinrichtung gemäß einem der Ansprüche 1 bis 4, wobei der Übertragungsführungskörper (5) einen Hohlraum besitzt, in dem der Bewegungsübertragungsdrahtkörper (2, 2a, 2b) geführt wird.

6. Auslenkbewegungsübertragungseinrichtung gemäß einem der Ansprüche 1 bis 4, wobei der Übertragungsführungskörper (5) ein Schienenelement ist, an dem der Bewegungsübertragungsdrahtkörper (2, 2a, 2b) geführt wird.

7. Auslenkbewegungsübertragungseinrichtung gemäß einem der Ansprüche 1 bis 6, wobei das Steuerelement (1) arretierbar ist, um eine Auslenkstellung des Steuerelements (1) zu arretieren.

8. Auslenkbewegungsübertragungseinrichtung Anspruch 7, wobei das Steuerelement (1) als Joystick vorgesehen ist, dessen Auslenkstellung durch eine Reibungsbremse arretierbar ist.

9. Auslenkbewegungsübertragungseinrichtung gemäß einem der Ansprüche 1 bis 8, wobei ein, zwei, drei, vier oder mehr Bewegungsübertragungsdrahtkörper (2, 2a, 2b) vorgesehen sind, von denen die ersten Enden gleichmäßig zueinander beabstandet äquatorial am Steuerelement (1) angelenkt sind und die entgegengesetzten zweiten Enden am distalen Endabschnitt des auszulenkenden Körpers in entsprechender Weise gleichmäßig zueinander beabstandet befestigt sind.

10. Endoskopbiegesteuerung mit einer Auslenkbewegungsübertragungseinrichtung gemäß einem der Ansprüche 1 bis 9, wobei der Übertragungsführungskörper (5) ein Katheterschlauch ist und der auszulenkende Körper (6) ein Biegeabschnitt ist.

11. Endoskop mit einer Endoskopbiegesteuerung gemäß Anspruch 10.

## Claims

1. Deflection movement transmission device having
- a control element (1) for establishing a deflection movement, wherein the control element (1) has a pivoting portion (11; 11') which is supported on a head portion (31; 31') of a base element (3) and for establishing a deflection movement is pivotable relative to the head portion (31; 31') of the base element (3);
- at least one movement transmission wire member (2, 2a, 2b) which is articulated on the pivoting portion (11; 11') of the control element (1);
- an elongate transmission guide member (5), the movement transmission wire member (2, 2a, 2b) being guided in the longitudinal direction of the former;
- and a flexible member (6) to be pivoted, which sits on that end of the transmission guide member (5) that is opposite the control element (1) and to which the movement transmission wire member (2, 2a, 2b) is attached so as to be spaced apart from the connection (61) to the transmission guide member (5);
- wherein the control element (1) is constructed as an activation lever which on the pivoting portion (11) has a base area (11A) that points towards the base body (3),
- wherein the head portion (31) of the base element (3) has an end face (31A) that points towards the control element (1),
- wherein the pivoting portion (11) of the control element (1) on the external side of the former has the articulation point (14) of the movement transmission wire member (2, 2a, 2b),
**characterized in that**
the base area (11A) of the pivoting portion (11) of the control element (1) and the interface (31A) of the head portion (31) of the base element (3) bulge in each case towards one another such that the base area (11A) and the end face (31A) are capable of rolling on one another.

2. Deflection movement transmission device according to Claim 1, wherein the head portion (31; 31') on that side that faces away from the control element (1) is integrally connected to a rod element (3) as the base element, and the rod element (3) for tensioning the movement transmission wire member (2, 2a, 2b) is axially movable in a rod element holder (4) from which the transmission guide member (5) extends.

3. Deflection movement transmission device according to Claim 2, wherein the transmission guide member (5) laterally of the rod element holder (4) extends from the longitudinal axis of the rod element (3) at a pre-defined angle.

4. Deflection movement transmission device according to one of Claims 1 to 3, wherein a first end of the movement transmission wire member (2, 2a, 2b) is articulated on the control element (1), and an opposite second end of the movement transmission wire member (2, 2a, 2b) is fastened to the distal end portion of the member (6) to be deflected.

5. Deflection movement transmission device according to one of Claims 1 to 4, wherein the transmission guide member (5) has a cavity in which the movement transmission wire member (2, 2a, 2b) is guided.

6. Deflection movement transmission device according to one of Claims 1 to 4, wherein the transmission guide member (5) is a rail element on which the movement transmission wire member (2, 2a, 2b) is guided.

7. Deflection movement transmission device according to one of Claims 1 to 6, wherein the control element (1) is lockable so as to lock a deflected position of the control element (1).

8. Deflection movement transmission device according to Claim 7, wherein the control element (1) is provided as a joystick, the deflected position of the latter being lockable by way of a friction brake.

9. Deflection movement transmission device according to one of Claims 1 to 8, wherein one, two, three, four, or more movement transmission wire members (2, 2a, 2b) are provided, the first ends of said movement transmission wire members (2, 2a, 2b) being articulated on the control element (1) in a uniformly mutually spaced-apart equatorial manner, the opposite second ends being fastened in a corresponding manner so as to be uniformly mutually spaced apart from one another to the distal end portion of the member to be deflected.

10. Endoscope bend control having a deflection movement transmission device according to one of Claims 1 to 9, wherein the transmission guide member (5) is a catheter tube and the member (6) to be deflected is a bending portion.

11. Endoscope having an endoscope bend control according to Claim 10.

## Revendications

1. Système de transmission d'un mouvement de déviation, avec
un élément de commande (1) pour l'exécution d'un mouvement de déviation, dans lequel l'élément de commande (1) comporte une partie pivotante (11; 11'), qui est appuyée sur une partie de tête (31; 31') d'un élément de base (3) et qui peut pivoter par rapport à la partie de tête (31; 31') de l'élément de base (3) pour l'exécution d'un mouvement de déviation,
au moins un corps de fil de transmission de mouvement (2, 2a, 2b), qui est articulé sur la partie pivotante (11; 11') de l'élément de commande (1),
un corps allongé de guidage de transmission (5), dans la direction longitudinale duquel le corps de fil de transmission de mouvement (2, 2a, 2b) est guidé,
un corps flexible à dévier (6), qui est monté sur l'extrémité du corps de guidage de transmission (5) opposée à l'élément de commande (1) et sur lequel le corps de fil de transmission de mouvement (2, 2a, 2b) est installé à distance de la liaison (61) avec le corps de guidage de transmission (5),
dans lequel l'élément de commande (1) est construit sous forme de levier d'actionnement, qui possède sur la partie pivotante (11) une face de pied (11A) tournée vers le corps de base (3),
dans lequel la partie de tête (31) de l'élément de base (3) possède une face frontale (31A) tournée vers l'élément de commande (1),
dans lequel la partie pivotante (11) de l'élément de commande (1) comporte sur son côté extérieur le point d'articulation (14) du corps de fil de transmission de mouvement (2, 2a, 2b),
**caractérisé en ce que** la face de pied (11A) de la partie pivotante (11) de l'élément de commande (1) et la face frontale (31A) de la partie de tête (31) de l'élément de base (3) sont respectivement bombées l'une vers l'autre, de telle manière que la face de pied (11A) et la face frontale (31A) puissent rouler l'une sur l'autre.

2. Système de transmission d'un mouvement de déviation selon la revendication 1, dans lequel la partie de tête (31; 31') est reliée sur le côté détourné de l'élément de commande (1) en une seule pièce à un élément de barre (3) en tant qu'élément de base et l'élément de barre (3) est déplaçable axialement dans un support d'élément de barre (4), à partir duquel le corps de guidage de transmission (5) s'étend, afin de tendre le corps de fil de transmission de mouvement (2, 2a, 2b).

3. Système de transmission d'un mouvement de déviation selon la revendication 2, dans lequel le corps de guidage de transmission (5) s'étend latéralement au support d'élément de barre (4) sous un angle prédéterminé par rapport à l'axe longitudinal de l'élément de barre (3).

4. Système de transmission d'un mouvement de déviation selon l'une quelconque des revendications 1 à 3, dans lequel une première extrémité du corps de fil de transmission de mouvement (2, 2a, 2b) est articulée sur l'élément de commande (1) et une deuxième extrémité opposée du corps de fil de transmission de mouvement (2, 2a, 2b) est fixée à la partie d'extrémité distale du corps à dévier (6).

5. Système de transmission d'un mouvement de déviation selon l'une quelconque des revendications 1 à 4, dans lequel le corps de guidage de transmission (5) comporte une cavité, dans laquelle le corps de fil de transmission de mouvement (2, 2a, 2b) est guidé.

6. Système de transmission d'un mouvement de déviation selon l'une quelconque des revendications 1 à 4, dans lequel le corps de guidage de transmission (5) est un élément de rail, sur lequel le corps de fil de transmission de mouvement (2, 2a, 2b) est guidé.

7. Système de transmission d'un mouvement de déviation selon l'une quelconque des revendications 1 à 6, dans lequel l'élément de commande (1) peut être bloqué, afin de bloquer une position déviée de l'élément de commande (1).

8. Système de transmission d'un mouvement de déviation selon la revendication 7, dans lequel l'élément de commande (1) est prévu sous forme de joystick, dont la position déviée peut être bloquée par un frein à friction.

9. Système de transmission d'un mouvement de déviation selon l'une quelconque des revendications 1 à 8, dans lequel il est prévu un, deux, trois, quatre ou davantage de corps de fil de transmission de mouvement (2, 2a, 2b), dont les premières extrémités sont articulées sur l'élément de commande (1) en position équatoriale à distance uniforme l'une de l'autre et les deuxièmes extrémités opposées sont fixées à la partie d'extrémité distale du corps à dévier d'une manière correspondante à distance uniforme l'une de l'autre.

10. Commande de la courbure d'un endoscope avec un système de transmission d'un mouvement de déviation selon l'une quelconque des revendications 1 à 9, dans lequel le corps de guidage de transmission (5) est un tuyau souple de cathéter et le corps à dévier (6) est une partie flexible.

11. Endoscope muni d'une commande de la courbure d'un endoscope selon la revendication 10.
